# EUROPEAN PATENT SPECIFICATION

(11) **EP 4 154 852 B1**
(45) Date of publication and mention of the grant of the patent: **10.07.2024**
(21) Application number: 20936340.7
(22) Date of filing: 21.05.2020
(51) Int. Cl.: A61F 9/007, A61B 34/32, A61B 17/34, A61B 34/20, A61B 34/30

(54) **SURGICAL TOOL RETRACTION MECHANISM**
RETRAKTIONSMECHANISMUS FÜR CHIRURGISCHES WERKZEUG
MÉCANISME DE RÉTRACTION D'OUTIL CHIRURGICAL

(43) Date of publication of application: 29.03.2023
(73) Proprietor: RIVERFIELD Inc., 1070052 Tokyo (JP)
(72) Inventor: SONODA, Koh-hei, Fukuoka-shi, Fukuoka 819-0395 (JP); NAKAO, Shintaro, Fukuoka-shi, Fukuoka 819-0395 (JP); TADANO, Kotaro, Tokyo 152-8550 (JP); FUKUDA, Tatsuhito, Tokyo 160-0017 (JP)
(74) Representative: adares Patent- und Rechtsanwälte Reininger & Partner GmbB
(86) International application number: PCT/JP2020/020201
(87) International publication number: WO 2021/234930

(56) References cited:
- WO-A1-2018/101265
- WO-A1-2018/101265
- WO-A1-2020/081434
- JP-A- 2014 514 016
- US-A1- 2012 211 006
- US-A1- 2012 211 006
- US-A1- 2015 342 695
- SHINTARO KIMURA, GAI YAMAUCHI, SHINJI KAWAKURA, KOTARO TADANO, YOSIKI KAIDU, SHINTARO NAKAO, KOHEI SONODA: "1P2-C06: Development of Endoscope Operation Robot Arm", 2018 LECTURE PROCEEDINGS; JSME CONFERENCE ON ROBOTICS AND MECHATRONICS; ROBOTICS MECHANICAL TRONICS LECTURE; 2018/06/02 - 2018/06/05, 25 December 2018 (2018-12-25), JP, pages 1P2-C06, XP009532385, ISSN: 2424-3124, DOI: 10.1299/jsmermd.2018.1P2-C06

## Description

### Technical Field

The present invention relates to a technology for quickly retracing a surgical device such as forceps or an endoscope from a surgical site.

### Background Art

Medical treatments using robots (manipulators) have recently been proposed in order to reduce the burden on the operators and save manpower in medical facilities. In the field of surgery, proposals have been made on surgical manipulator systems for operators to treat patients by operating remotely-controllable surgical manipulators.

For example, Non-Patent Literature 1 teaches an intraocular endoscope manipulator robot that inserts an endoscope into an eyeball in a state in which a brake of an arm is off so that joints of the arm with three degrees of freedom of the arm become passive, and the applies the brake. Because an eyeball of a patient may move during an operation on the eyeball, the aforementioned manipulator robot includes a mechanism for disabling the brake when the patient moves so that the joints become passive, and retracting the endoscope from the eyeball by the arm position.
Patent Literature 1 describes a surgical tool retraction mechanism comprising: an implement retraction base that holds a surgical tool; guide rails; a locking pin; and a locking pin retraction solenoid which pulls, when actuated, the locking pin out, which releases the implement retraction base, retracting both a needle and a dilator that releases the locked state of the locking part on the basis of a signal from the outside and that retracts the implement retraction base. Patent Literature 2 describes a surgical instrument unit comprising a surgical instrument and a support device for supporting it, wherein the surgical instrument can include an endoscope, an electric scalpel, forceps, and an examination electrode. The support device includes a support portion having a hollow portion. On the outer peripheral surface of the support device, a solenoid for projecting or retracting a stopper toward the center in the radial direction is provided inside the hollow portion, wherein the solenoid may project the stopper when energized and retract the stopper when not energized, or retract the stopper when energized and project the stopper when not energized. Further, a spring is inserted in the hollow portion.
Patent Literature 3 describes a device providing springcontrolled application of the precise force and velocity needed for a piercing needle, wherein to prime the device, the user lifts a plunger, which pulls an inner needle into an outer needle, and in this position the inner needle locks in place through a spring-locking mechanism and the inner needle is released by pressing a release such as a lever, which unlocks the spring-locking mechanism to allow a primary spring and the plunger to force the piercing needle into the vein. A primary compression spring is positioned along a shaft of the plunger. Release lever is positioned on and is rotatable around the axis of a cylindrical hub and is held in place by secondary compression spring, which by default holds release lever in the locked position. When the user pulls the plunger up, the release lever is displaced, the secondary compression spring is compressed, and the release lever rotates around its cylindrical pivot.
Patent Literature 4 describes a multi-force sensing instrument including a tool, a strain sensor, at least one of a second strain sensor or a torque-force sensor, and a signal processor configured to communicate with the strain sensor and the at least one of the second strain sensor or the torque-force sensor to receive detection signals therefrom.

### Related Art List

### Non-Patent Literature

Non-Patent Literature 1: Shintaro KIMURA et al., "Development of Intraocular Endoscope Manipulator", proceedings of JSME annual Conference on Robotics and Mechatronics (Robomec), The Japan Society of Mechanical Engineers, 2018, 2018(0), 1P2-C06

### Patent Literature

Patent Literature 1: US 2012/211006 A1
Patent Literature 2: WO 2018/101265 A1
Patent Literature 3: WO 2020/081434 A1
Patent Literature 4: US 2015/342695 A1

### Summary of Invention

### Technical Problem

Because, however, the aforementioned robot retracts the endoscope by moving the arm, the direction in which the endoscope is retracted from the eyeball is not linear, and a load is thus applied to the eyeball during retraction.

The present invention has been made in view of the aforementioned circumstances, and an exemplary object thereof is to provide a novel technology for reducing a load applied to a surgical site while a surgical device is retracted from the surgical site.

### Solution to problem

A surgical device retracting mechanism according to an aspect of the present invention includes: a holder for holding a surgical device; a guiding member for guiding the holder to linearly advance and retract; a locking member for locking the holder in a state in which the surgical device is inserted in a surgical site; and a retracting unit for releasing a locking state of the locking member and retracting the holder in accordance with a signal from outside the retracting unit.

This aspect allows a surgical device linearly inserted into a surgical site along the guiding member to be retracted in parallel with the inserting direction of the surgical device. As a result, the load applied to the surgical site during retraction of the surgical device is reduced.

The retracting unit includes: a first force generating member for generating a first force in a retracting direction of the holder in a state in which the holder is locked; a second force generating member for generating a second force for releasing the locking state of the locking member; and a third force generating member for maintaining the locking state of the locking member against the second force. This achieves holding and retraction of a surgical device by adjusting the forces generated by the respective force generating members by a simple structure.

The locking member is a turnable member that is turnable about a fulcrum, and the turnable member has a first end coming in contact with the holder and a second end on which the third force acts in the locking state. This allows the third force generating member for generating the third force to be placed at a position away from the holder, which increases the flexibility of layout of respective components without making the structure complicated.

The third force generating member is a solenoid for actuating the second end of the turnable member while power supply to the solenoid is on. The second force generating member may be a compression spring, and the third force generating member may be a pull-type solenoid for pulling the second end of the turnable member while power supply to the pull-type solenoid is on. Alternatively, the second force generating member may be a tension spring, and the third force generating member may be a push-type solenoid for pushing the second end of the turnable member while power supply to the push-type solenoid is on. As a result, in such an emergency in which power supply to the pull-type solenoid or the push-type solenoid is cut off owing to a power failure or a device failure, for example, the surgical device is quickly retracted from the surgical site without requiring special control.

The compression spring of the retracting unit may have a spring constant set so that the locking state of the locking member is released while power supply to the pull-type solenoid is off. This allows generation of the second force by the restoring force of the compression spring without using power.

The first force generating member may be a tension spring. This allows generation of the first force by the restoring force of the tension spring without using power.

The first force generating member may be a tension spring. This allows generation of the first force by the restoring force of the tension spring without using power.

The surgical device held by the holder may be an endoscope to be inserted into an eyeball. This allows an endoscope inserted in such a soft and delicate surgical site as an eyeball to be quickly retracted in an emergency while reducing the load applied to the eyeball.

A sensor for detecting a movement of an eyeball or a movement of a head may further be included. The retracting unit may be configured to release the locking state of the locking member in accordance with a signal in response to the movement of the eyeball or the movement of the head detected by the sensor. This allows the endoscope held in a state inserted in an eyeball to be quickly retracted before a great load is applied to the eyeball by the movement of the position of the eyeball.

### Advantageous Effects of Invention

According to the present invention, the load applied to a surgical site when a surgical device is retracted from the surgical site is reduced.

### Brief Description of Drawings

FIG. 1 is a side view illustrating an outline structure in a state in which an endoscope is inserted into an eyeball by a surgical device retracting mechanism according to an embodiment.
FIG. 2 is a side view illustrating an outline structure in a state in which the endoscope is retracted from the eyeball by the surgical device retracting mechanism according to an embodiment.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. Thu

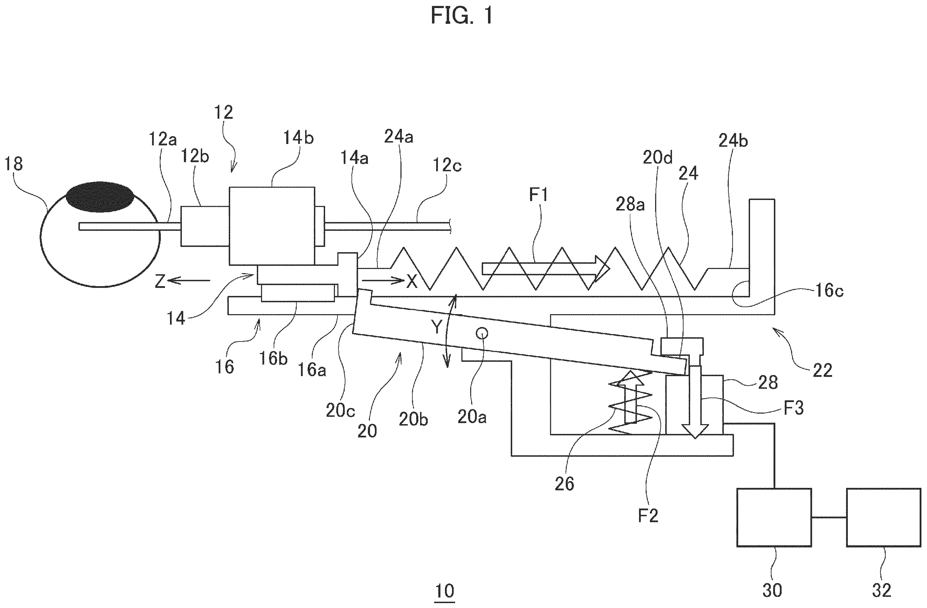

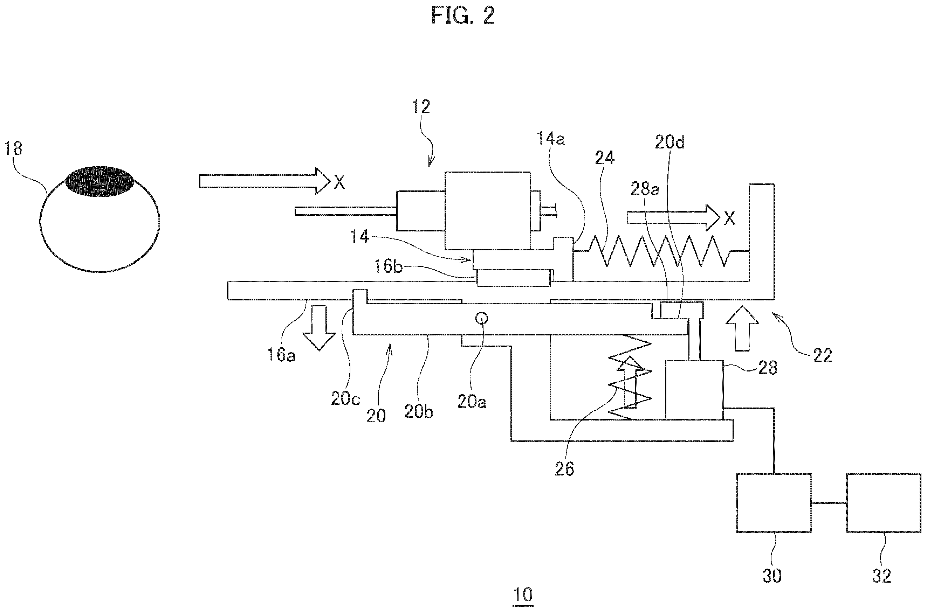

FIG. 1 is a side view illustrating an outline structure in a state in which an endoscope is inserted into an eyeball by a surgical device retracting mechanism according to an embodiment. FIG. 2 is a side view illustrating an outline structure in a state in which the endoscope is retracted from the eyeball by the surgical device retracting mechanism according to an embodiment.

A surgical device retracting mechanism 10 illustrated in FIG. 1 includes a holder 14 for holding an endoscope 12, which is a surgical device, a guiding member 16 for guiding the holder 14 to linearly advance and retract, a locking member 20 for locking the position of the holder 14 in a state in which the endoscope 12 is inserted in an eyeball 18, which is a surgical site, and a retracting unit 22 for releasing the locking state of the locking member 20 and retracting the holder 14 in accordance with a signal from outside.

The endoscope 12 includes a fiber-like insertion portion 12a having a diameter of about 0.5 to 1.2 mm to be inserted into an eyeball 18, and a grip 12b, a cable 12c. The grip 12b is held by an adapter 14b of the holder 14. The guiding member 16 includes a linear guide 16a, and a stage 16b configured to slide along the linear guide 16a.

The retracting unit 22 includes a tension spring 24 that is a first force generating member for generating a first force F1 in a direction X in which the holder 14 is to be retracted in a state in which the holder 14 is locked, a compression spring 26 that is a second force generating member for generating a second force F2 for releasing the locking state of the locking member 20, and a pull-type solenoid 28 that is a third force generating member for generating a third force F3 for maintaining, against the second force F2, the locking state of the locking member 20.

A first end 24a of the tension spring 24 is fixed to an end 14a in the retracting direction of the holder 14. A second end 24b of the tension spring 24 is fixed to an end 16c of the linear guide 16a. The locking member 20 is a rod-like turnable member 20b configured to turn about a fulcrum 20a in a Y direction. The turnable member 20b has a first end 20c that comes in contact with the end 14a of the holder 14 and a second end 20d on which the third force F3 acts in the locking state.

The pull-type solenoid 28 pulls the second end 20d of the turnable member 20b while power is on. Specifically, the pull-type solenoid 28 has a hook-like catching portion 28a at an end of a plunger thereof. The catching portion 28a fixes the second end 20d of the turnable member 20b in a state in which the second end 20d is pulled downward.

The compression spring 26 of the retracting unit 22 according to the embodiment has a spring constant and a length set so that the locking state of the locking member 20 is released while the pull-type solenoid 28 is powered off. In addition, the retracting unit 22 further includes a control unit 30 for controlling power supply to the pull-type solenoid 28, and a sensor 32 for detecting the movement of an eyeball 18 or the movement of a head.

Next, an operation of immediately retracting the endoscope 12 from an eyeball 18 in an X direction will be explained with reference to FIG. 2. As illustrated in FIG. 2, when the pull-type solenoid 28 is powered off, the third force F3 by which the catching portion 28a of the pull-type solenoid 28 has pulled the second end 20d of the turnable member 20b downward is eliminated. As a result, the second end 20d is pushed upward and the first end 20c, which is located on a side opposite the second end 20d with respect to the fulcrum 20a, is pushed downward by the restoring force of the compression spring 26, and the locking of the holder 14 by the locking member 20 is thus released.

As a result, the restoring force of the tension spring 24 with the first end 24a fixed to the end 14a of the holder 14 moves the holder 14 in the X direction, and the endoscope 12 is retracted from the eyeball 18 in the X direction.

As described above, the surgical device retracting mechanism 10 according to the embodiment allows the endoscope 12 linearly inserted into an eyeball 18 that is the surgical site along the linear guide 16a to be retracted in parallel (in the X direction) with the inserting direction Z of the endoscope 12. As a result, the load applied to the eyeball 18 while the endoscope 12 is retracted from the eyeball 18 is reduced.

In addition, the surgical device retracting mechanism 10 achieves holding and retraction of the endoscope 12 by adjusting the forces generated by the tension spring 24, the compression spring 26, and the pull-type solenoid 28 by a simple structure.

In addition, in the surgical device retracting mechanism 10, because the pull-type solenoid 28 that requires power supply and a relatively large space may be placed at a position away from the holder 14, the flexibility of layout of respective components is increased without making the structure complicated.

Furthermore, in such an emergency in which power supply to the pull-type solenoid 28 is cut off owing to a power failure or a device failure, for example, the surgical device retracting mechanism 10 is capable of quickly retracting the endoscope 12 from an eyeball 18 without requiring special control. In addition, the surgical device retracting mechanism 10 is capable of generating the second force F2 by the restoring force of the compression spring 26 without using power, and similarly capable of generating the first force F1 by the restoring force of the tension spring 24 without using power.

Furthermore, the surgical device retracting mechanism 10 is capable of quickly retracting the endoscope 12 inserted in such a soft and delicate surgical site as the eyeball 18 in an emergency while reducing the load applied to the eyeball 18.

In addition, the control unit 30 of the retracting unit 22 according to the embodiment is configured to release the locking state of the locking member 20 by controlling current to turn off power supply to the pull-type solenoid 28 in accordance with a signal in response to a movement of the eyeball or the like detected by the sensor 32. This allows the endoscope 12 held in a state inserted in the eyeball 18 to be quickly retracted before a large load is applied to the eyeball 18 by the movement of the position of the eyeball 18.

While the retracting unit 22 according to the embodiment is a combination of the compression spring 26, which is the second force generating member, and the pull-type solenoid 28, which is the third force generating member, a retracting unit may be configured as a combination of a tension spring, as a second force generating member, for pulling the turnable member 20b upward and a push-type solenoid, as a third force generating member, for pushing the second end 20d of the turnable member 20b downward when power is supplied to the push-type solenoid.

While the present invention has been described above with reference to the embodiment, the present invention is limited by the scope of independent claim 1.

### Industrial Applicability

The present invention is applicable to a technology for quickly retracting a surgical device such as forceps or an endoscope from a surgical site.

### Reference Signs List

- F1: first force
- F2: second force
- F3: third force
- 10: surgical device retracting mechanism
- 12: endoscope
- 14: holder
- 16: guiding member
- 18: eyeball
- 20: locking member
- 20a: fulcrum
- 20b: turnable member
- 20c: first end
- 20d: second end
- 22: retracting unit
- 24: tension spring
- 26: compression spring
- 28: pull-type solenoid
- 30: control unit
- 32: sensor

## Claims

1. A surgical device retracting mechanism (10) comprising:
a holder (14) for holding a surgical device;
a guiding member (16) for guiding the holder (14) to linearly advance and retract;
a locking member (20) for locking the holder (14) in a state in which the surgical device is inserted in a surgical site; and
a retracting unit (22) for releasing a locking state of the locking member (20) and retracting the holder (14) in accordance with a signal from outside the retracting unit (22), wherein the retracting unit (22) includes:
a first force generating member for generating a first force (F1) in a retracting direction of the holder (14) in a state in which the holder (14) is locked;
a second force generating member for generating a second force (F2) for releasing the locking state of the locking member; and
a third force generating member for maintaining the locking state of the locking member against the second force (F2)
**characterized in that**
the locking member (20) is a turnable member (20b) that is turnable about a fulcrum (20a), and
the turnable member (20b) has a first end (20c) coming in contact with the holder (14) and a second end (20d) on which the third force (F3) acts in the locking state the third force generating member is a solenoid (28) for actuating the second end (20d) of the turnable member (20b) while power supply to the solenoid (28) is on

2. The surgical device retracting mechanism (10) according to claim 1, wherein
the second force generating member is a compression spring (26), and
the third force generating member is a pull-type solenoid (28) for pulling the second end (20d) of the turnable member (20b) while power supply to the pull-type solenoid (28) is on.

3. The surgical device retracting mechanism (10) according to claim 2, wherein
the compression spring (26) of the retracting unit (22) has a spring constant set so that the locking state of the locking member (20) is released while power supply to the pull-type solenoid (28) is off.

4. The surgical device retracting mechanism (10) according to any one of claims 2 to 3, wherein
the first force generating member is a tension spring (24) .

5. The surgical device retracting mechanism (10) according to any one of claims 1 to 4, wherein
the surgical device held by the holder (14) is an endoscope (12) to be inserted into an eyeball (18).

6. The surgical device retracting mechanism (10) according to any one of claims 1 to 5, further comprising:
a sensor (32) for detecting a movement of an eyeball (18) or a movement of a head, wherein
the retracting unit (22) is configured to release the locking state of the locking member (20) in accordance with a signal in response to the movement of the eyeball (18) or the movement of the head detected by the sensor (32).

## Patentansprüche

1. Chirurgievorrichtung-Rückzugsmechanismus (10), enthaltend:
einen Halter (14) zum Halten einer Chirurgievorrichtung;
ein Führungselement (16) zum Führen des Halters (14) zum linearen Vorfahren und Zurückziehen;
ein Festsetzelement (20) zum Festsetzen des Halters (14) in einem Zustand, in dem die Chirurgievorrichtung in eine chirurgische Stelle eingeführt ist; und
eine Rückzugseinheit (22) zum Lösen eines Festsetzzustandes des Festsetzelements (20) und Zurückfahren des Halters (14) gemäß einem Signal von außerhalb der Rückzugseinheit (22),
wobei die Rückzugseinheit (22) einschließt:
ein erstes Krafterzeugungselement zum Erzeugen einer ersten Kraft (F1) in einer Rückzugsrichtung des Halters (14) in einem Zustand, in dem der Halter (14) festgesetzt ist;
ein zweites Krafterzeugungselement zum Erzeugen einer zweiten Kraft (F2) zum Freigeben des Festsetzzustands des Festsetzelements; und
ein drittes Krafterzeugungselement zur Aufrechterhaltung des Festsetzzustandes des Festsetzelements gegenüber der zweiten Kraft (F2)
**dadurch gekennzeichnet, dass**
das Festsetzelement (20) ein drehbares Element (20b) ist, das um einen Drehpunkt (20a) drehbar ist, und
das drehbare Element (20b) ein erstes Ende (20c), das mit dem Halter (14) in Kontakt kommt, und ein zweites Ende (20d) aufweist, auf das die dritte Kraft (F3) im Festsetzzustand wirkt, das dritte Krafterzeugungselement ein Solenoid (28) zum Betätigen des zweiten Endes (20d) des drehbaren Elements (2b) ist, während eine Stromversorgung des Solenoids (28) eingeschaltet ist.

2. Chirurgievorrichtung-Rückzugsmechanismus (10) nach Anspruch 1, wobei
das zweite Krafterzeugungselement eine Druckfeder (26) ist, und
das dritte Krafterzeugungselement ein Zug-Typ-Solenoid (28) zum Ziehen des zweiten Endes (20d) des drehbaren Elements (20b) ist, während die Stromversorgung des Solenoids (28) eingeschaltet ist.

3. Chirurgievorrichtung-Rückzugsmechanismus (10) nach Anspruch 2, wobei
die Druckfeder (26) der Rückzugseinheit (22) eine Federkonstante aufweist, die so eingestellt ist, dass der Festsetzzustand des Festsetzelements (20) freigegeben wird, während die Stromversorgung des Zug-Typ-Solenoiden (28) ausgeschaltet ist.

4. Chirurgievorrichtung-Rückzugsmechanismus (10) nach einem der Ansprüche 2 bis 3, wobei
das erste Krafterzeugungselement eine Zugfeder (24) ist.

5. Chirurgievorrichtung-Rückzugsmechanismus (10) nach einem der Ansprüche 1 bis 4, wobei
die Chirurgievorrichtung, die von dem Halter (14) gehalten wird, ein Endoskop (12) ist, das in einen Augapfel (18) einzuführen ist.

6. Chirurgievorrichtung-Rückzugsmechanismus (10) nach einem der Ansprüche 1 bis 5, ferner enthaltend:
einen Sensor (32) zum Erfassen einer Bewegung eines Augapfels (18) oder einer Bewegung eines Kopfes, wobei
die Rückzugseinheit (22) konfiguriert ist, den Festsetzzustand des Festsetzelements (20) gemäß einem Signal als Antwort auf die Bewegung des Augapfels (18) oder die Bewegung des Kopfes freizugeben, die von dem Sensor (32) erfasst wird.

## Revendications

1. Un mécanisme de rétraction pour un dispositif chirurgical (10) comprenant :
un support (14) pour tenir un dispositif chirurgical ;
un élément de guidage (16) pour guider le support (14) pour avancer et rétracter linéairement ;
un élément de blocage (20) pour bloquer le support (14) dans un état dans lequel le dispositif chirurgical est inséré dans un site chirurgical ; et
une unité de rétraction (22) permettant de libérer un état de blocage de l'élément de blocage (20) et de rétracter le support (14) conformément à un signal provenant de l'extérieur de l'unité de rétraction (22),
dans lequel l'unité de rétraction (22) comprend :
un premier élément générateur de force pour générer une première force (F1) dans une direction de rétraction du support (14) dans un état dans lequel le support (14) est bloqué ;
un deuxième élément générateur de force pour générer une deuxième force (F2) pour libérer l'état de blocage de l'élément de blocage ; et
un troisième élément générateur de force pour maintenir l'état de blocage de l'élément de blocage contre la deuxième force (F2)
**caractérisé en ce que**
l'élément de blocage (20) est un élément tournant (20b) qui est tournant autour d'un point d'appui (20a), et
l'élément tournant (20b) a une première extrémité (20c) qui entre en contact avec le support (14) et une deuxième extrémité (20d) sur laquelle la troisième force (F3) agit à l'état de blocage, le troisième élément générateur de force est un solénoïde (28) pour actionner la deuxième extrémité (20d) de l'élément tournant (20b), lorsque l'alimentation électrique du solénoïde (28) est activée.

2. Le mécanisme de rétraction pour un dispositif chirurgical (10) selon la revendication 1, dans lequel
le deuxième élément générateur de force est un ressort de compression (26), et
le troisième élément générateur de force est un solénoïde de type traction (28) pour tirer la deuxième extrémité (20d) de l'élément tournant (20b) lorsque l'alimentation électrique du solénoïde de type traction (28) est activée.

3. Le mécanisme de rétraction pour un dispositif chirurgical (10) selon la revendication 2, dans lequel
le ressort de compression (26) de l'unité de rétraction (22) a une constante de ressort réglée de sorte que l'état de blocage de l'élément de blocage (20) est libéré lorsque l'alimentation électrique du solénoïde de type traction (28) est coupée.

4. Le mécanisme de rétraction pour un dispositif chirurgical (10) selon l'une quelconque des revendications 2 à 3, dans lequel
le premier élément générateur de force est un ressort de tension (24).

5. Le mécanisme de rétraction pour un dispositif chirurgical (10) selon l'une quelconque des revendications 1 à 4, dans lequel
le dispositif chirurgical tenu par le support (14) est un endoscope (12) à insérer dans un globe oculaire (18).

6. Le mécanisme de rétraction pour un dispositif chirurgical (10) selon l'une quelconque des revendications 1 à 5, comprenant en outre :
un capteur (32) pour détecter un mouvement d'un globe oculaire (18) ou un mouvement d'une tête, dans lequel
l'unité de rétraction (22) est configurée pour libérer l'état de blocage de l'élément de blocage (20) conformément à un signal en réponse au mouvement du globe oculaire (18) ou au mouvement de la tête détecté par le capteur (32).
